# EUROPEAN PATENT APPLICATION

(11) **EP 2 289 343 A1**
(43) Date of publication of application: **02.03.2011**
(21) Application number: 09166698.2
(22) Date of filing: 29.07.2009
(51) Int. Cl.: A23C 9/152, A23L 1/30, A61K 31/7048

(54) **Flavanones-containing food compositions**

(71) Applicant: Nestec S.A., 1800 Vevey (CH)
(72) Inventor: Hoebler, Pascaline, 3286, MUNTELIER (CH)
(74) Representative: Chautard, Cécile

(57) **Abstract**

The present invention relates to food products comprising flavanones. In particular, it relates to food products comprising hesperidin having improved stability. The present invention also concerns processes for the manufacturing of food products comprising said flavanones, especially hesperidin and to the use of the food products in the manufacture of compositions for the improvement of bone and skin health. Said flavanones are subjected to heating in water to a temperature of at least 138°C.

## Description

### Field of the present invention

The present invention relates to food products comprising flavanones. In particular, it relates to food products comprising hesperidin and having improved stability. The present invention also concerns processes for the manufacturing of food products comprising said flavanones, especially hesperidin and to the use of the food products in the manufacture of compositions for the improvement of bone and skin health.

### Background of the invention

Hesperidin is a known flavonoid compound which has a number of health benefits associated to its uptake. These benefits include antioxidant properties, antiinflammatory properties, cardio-protective and lipid lowering effect, anti-microbial activity, anti-carcinogenic activity, UV protective activity etc. Medicinal applications of hesperidin are for example disclosed in EP 1 536 806.

Commercially available hesperidin is difficult to incorporate into foods due to its poor water or oil solubility. Due to these difficulties, it has only been possible so far to include hesperidin in products where sedimentation is not an issue, such as food supplements in the form of powders, or chewing gum for example, or in liquid products where the hesperidin concentration is very low.

Such liquid products containing flavanones such as hesperidin are known from the prior art. For instance, US 3,615,717 describes a liquid milk product which comprises hesperidin in an amount of at most 0.04% in order to inhibit staling.

In US 2006/0105089 a refined peel juice comprising at most 70ppm hesperidin is described.

Such low concentrations of flavanones as are described in the liquid products of the prior art do not impact on the issue of storage stability or sedimentation.

A suggested approach to incorporate hesperidin in higher amounts into a liquid product includes wet, dry and jet milling of hesperidin prior to integration in the liquid product. This approach is however very costly and requires investment in a specific processing equipment.

Another approach is to use stabilising agents and/or increase the viscosity of the liquid product to prevent rapid sedimentation of hesperidin. Although this is applicable to liquid products, at high concentration at which it could stabilize hesperidin, it would affect the sensorial properties of the product, e.g. viscosity. This approach is also not easily applicable for the manufacture of powder products to be reconstituted in a liquid.

### Object of the present invention

It is therefore an object of the present invention to improve the stability of liquid food products or reconstituted powders comprising flavanones, especially hesperidin and to provide a wider range of food products comprising said flavanones.

### Summary of the invention

This object is solved by means of the independent claims. The dependent claims further serve to develop the central idea of the invention.

An aspect of the invention concerns a process for the manufacture of a powder, liquid or semi-liquid food product comprising flavanones, comprising the steps of:
a. Heating said flavanone in water to a temperature of at least 138°C, preferably 140-160°C, for 1 to 60 seconds and
b. Incorporating said flavanone to the food product.

Another aspect of the invention relates to a process for the manufacture of powder, liquid or semi-liquid food product comprising flavanones, especially hesperidin comprising the step of heating a liquid or semi-liquid food product comprising said flavanone in an amount of 0.05-5% to a temperature of at least 138°C, preferably 138-160°C, most preferred 140-150°C for 1-60s.

Food products obtainable by these processes also form part of the invention.

In another aspect, the invention provides a process for improving the stability of flavanones in a liquid or semi-liquid food product comprising the steps of:
a. Heating said flavanone in water to a temperature of at least 138°C, preferably 140-160°C, for 1 to 60 seconds and
b. Incorporating said flavanone to the food product
or
heating said food product in the presence of said flavanone to a temperature of at least 138°C, preferably 138-160°C, most preferred 140-150°C for 1-60s.

A food product comprising flavanones in an amount of 0.05-5%, wherein the flavanone particles are obtained by heating said flavanone at a temperature of at least 138°C, preferably 138-160°C, most preferred 140-150°C for 1-60s is also part of the present invention.

Furthermore, the invention also pertains to the use of a food product according to any of claims 10 to 13 for the manufacture of a composition for the improvement of bone and skin health.

In a further aspect, the invention relates to a process for preparing a liquid or semi-liquid food product, comprising the step of reconstituting the food product of claim 9 or 12 in a liquid such that the concentration of flavanone in the liquid or semi-liquid product is between 0.05-0.5%, preferably 0.05-0.3%, more preferably 0.05-0.1%.

Finally, a process for producing stabilised flavanones comprising the step of heating said flavanone in water to a temperature of at least 138°C, preferably 140-160°C, for 1 to 60 seconds and the flavanone obtainable by such process are also part of the invention.

### Figures

The present invention is further described hereinafter with reference to some of its embodiments shown in the accompanying drawings in which:
- Fig. 1 is a flow chart illustrating the heat treatment process according to the invention (MSK: skimmed milk solids, TS: total solids)
- Fig. 2 shows microscopy images of hesperidin in water before heat treatment (fig. 2.1), after heat treatment at 140°C for 10s (fig. 2.2), after heat treatment at 140°C for 60s (fig. 2.3), after heat treatment at 160°C for 10s (fig. 2.4).
- Fig. 3 shows the particle size distribution of hesperidin in water before heat treatment (fig. 3.1), after heat treatment at 140°C for 10s (fig. 3.2), after heat treatment at 140°C for 60s (fig. 3.3), after heat treatment at 160°C for 10s (fig. 3.4).
- Fig. 4.1 shows pictures taken 5 minutes after preparation of samples of skimmed milk containing commercial hesperidin in an amount of 0.1 % and 0.5% respectively (samples 1 and 3) and skimmed milk containing heat treated hesperidin according to the invention (samples 2 and 4).
- Fig. 4.2 shows pictures taken 12 hours after preparation of samples of skimmed milk containing commercial hesperidin in an amount of 0.1 % and 0.5% respectively (samples 1 and 3) and skimmed milk containing heat treated hesperidin according to the invention (samples 2 and 4).
- Fig. 5 shows the particle size distribution of commercial hesperidin in water (fig. 5.1), of dry milled hesperidin in water (fig. 5.2) and of heat treated hesperidin in water (fig. 5.3).
- Fig. 6.1 shows pictures of samples taken 5 minutes after preparation of commercial hesperidin in reconstituted milk (sample 1), of dry milled hesperidin in reconstituted milk (sample 2) and of heat treated hesperidin in reconstituted milk (sample 3).
- Fig. 6.2 shows pictures of samples taken 12 hours after preparation of commercial hesperidin in reconstituted milk (sample 1), of dry milled hesperidin in reconstituted milk (sample 2) and of heat treated hesperidin in reconstituted milk (sample 3).

### Detailed description of the invention

The invention is described in the following by reference to hesperidin as a preferred embodiment of the invention. However, the invention equally relates to other flavanones such as naringin, eriocitrin, etc. Preferably, the invention relates to flavanones which are water insoluble. By "water insoluble" is meant that the flavanone does not dissolve at room temperature (e.g. 20°C) in water having a neutral pH (e.g. about 7).

In the following the invention is described by reference to food products as a preferred embodiment. However, the invention also relates to products for topical application or medicinal products.

The present invention relates to a process for the manufacture of a food product comprising a flavanone, especially hesperidin. The hesperidin obtained by carrying out the process step is stabilised which constitutes an advantage for the food product in terms of storage stability and organoleptic properties.

It has been found by the inventors thus that a process comprising the step of heating a flavanone in water to a temperature of at least 138°C, preferably 140-160°C for 1 to 60 seconds produces stabilised flavanone. The flavanone obtainable by the process is also part of the invention.

By "stabilised" is meant that, compared to hesperidin which has not undergone the processes of the invention, the hesperidin in the food product does not easily sediment out of solution. The hesperidin in the stabilised form of the invention confers stability upon storage to the product containing it.

According to the invention, a process for the manufacture of a powder, liquid or semi-liquid food product comprising flavanones comprises the step of heating said flavanone in water to a temperature of at least 138°C, preferably 140-160°C for 1 to 60 seconds. The flavanone which has been heat treated is then incorporated to the food product.

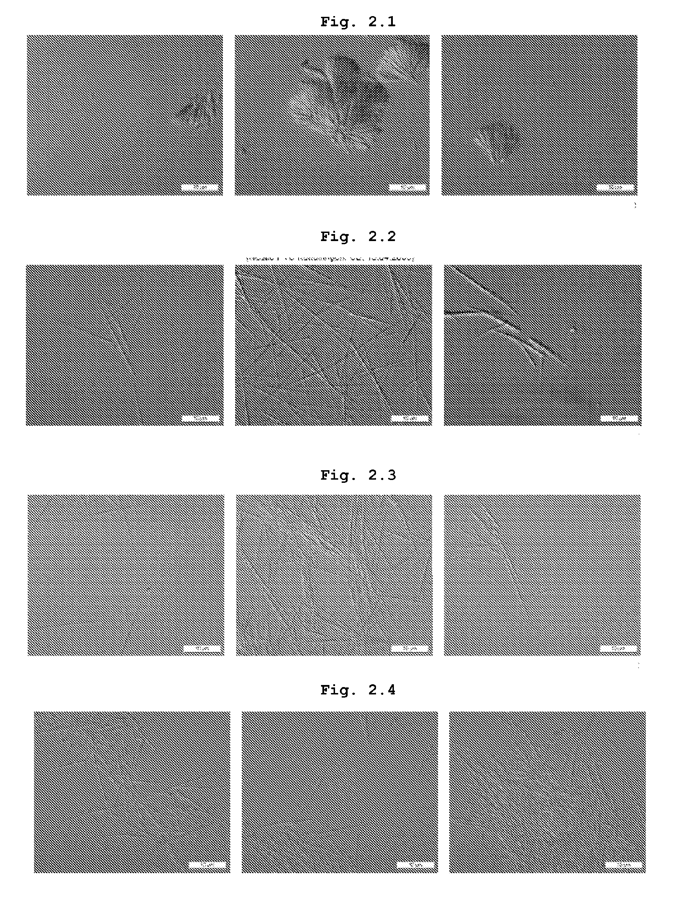

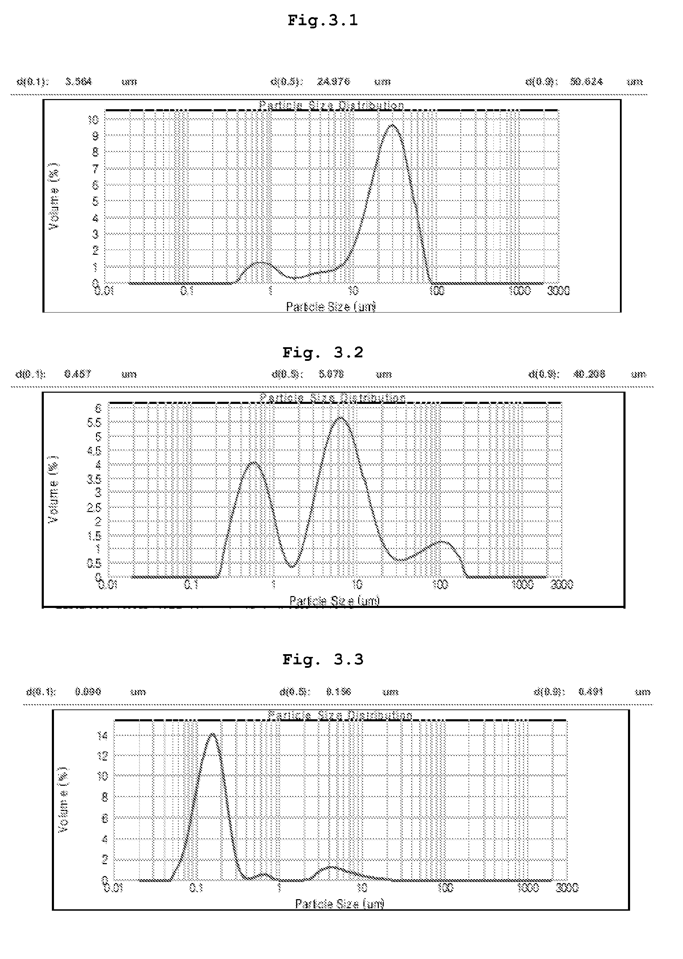

Upon heat treatment, the flavanone undergoes morphological changes. Fig. 2.1 shows hesperidin crystals which have not undergone a heat treatment. In comparison, Figures 2.2, 2.3, 2.4 show that upon heat treatment the initial crystals change shape and become needle-like. Thus, the hesperidin is in the form of needles which are not agglomerated. Additionally, the particle size distribution is reduced compared to hesperidin which has not undergone a heat treatment (cf. figures 3.1 to 3.4). In a particular embodiment, at least 80% of the cumulative distribution of hesperidin particle is below 20 microns.

The particle size distribution is observed using a microscopy optic LEICA DMR (mode Differential Interference Contrast and polarisation). The particle size distribution is measured by laser diffraction using a Malvern Mastersizer 2000.

Another method of the invention for the manufacture of a powder, a liquid or semi-liquid food product comprising flavanones comprises the step of heating a liquid or semi-liquid food product comprising said flavanone in an amount of 0.05-0.5% to a temperature of at least 138°C, preferably 140-160°C, most preferred 140-150°C for 1-60s, preferably 10-30s.

In this process of the invention, hesperidin is added to the liquid or semi-liquid food product. Hesperidin is preferably added as an aqueous solution comprising 0.1-30%, more preferably 0.1-20 wt%, even more preferably 5% hesperidin. The liquid or semi-liquid food product is then preferably preheated to 70°C, followed by a heat treatment at preferably about 140°C. This heat treatment preferably lasts for about 1 to 60 seconds, more preferably about 10 seconds.

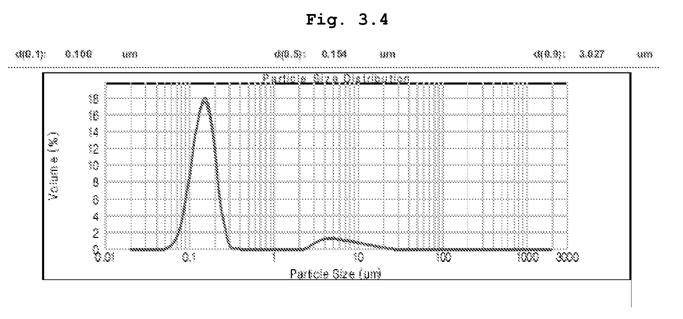

Again, as a result of the heat treatment, the hesperidin particles undergo a change of size and morphology as shown in figures 2 and 3.

The mixture of heat treated hesperidin and food product may then be further processed depending on the desired end product. Further processing steps may include pH adjustment, homogenisation, pasteurisation, preheating, cooling, filling, drying, spray-drying etc.

In an embodiment of the present invention, the liquid or semi-liquid food product is milk-based. In this embodiment, the food product to which hesperidin is added preferably contains a stabilising agent. Stabilising agents may be selected from alginates, carrageenan, cellulose gum, guar gum, microcrystalline cellulose, pectin, xanthan, etc. Preferably, it is cellulose gum and microcrystalline cellulose.

Without wishing to be bound by theory, it is thought that a small portion of milk protein in liquid skimmed milk or reconstituted skimmed milk powder can sediment thus leading to sedimentation of some hesperidin. To prevent any protein sedimentation and hence hesperidin sedimentation, a stabilising agent can be used.

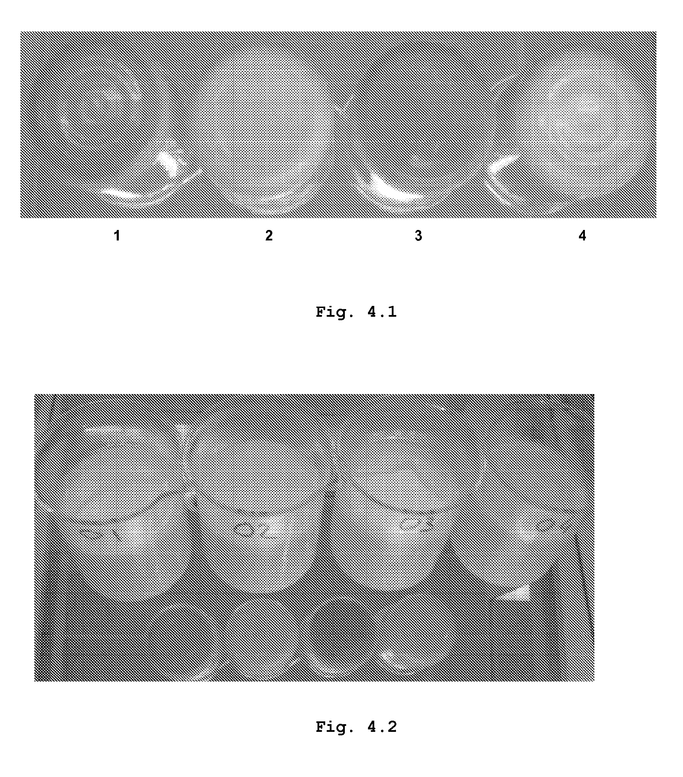

Additionally, as described in example 3 and fig. 4.1 and 4.2, it was confirmed that hesperidin is stabilised by the heat treatment according to the invention. Indeed, simply adding hesperidin which has not been subjected to a heat treatment to a milk-based product, even one containing a stabilising agent, leads to a product which is unstable to storage due to hesperidin sedimentation.

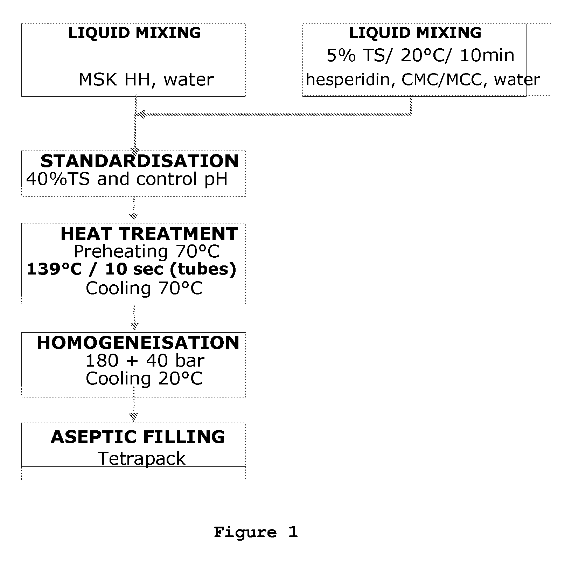

In a preferred embodiment of the method of the invention depicted in the flow chart of figure 1, a solution of hesperidin and stabilising agent (CMC/MCC) in water is added to skimmed milk. The mixture is then standardised to a total solids content of about 10% and the pH is checked and adjusted if necessary. The mixture is then preheated to 70°C, heated to 140°C for 10 seconds, then two-stage homogenised at a pressure of 180 bars and 40 bars. The mixture is then cooled giving a final skimmed milk product comprising hesperidin. The skimmed milk is stable at room temperature for at least 3 months without any signs of hesperidin precipitation.

The liquid or semi-liquid food product according to the invention may be any of skimmed milk, partially skimmed milk, filled milk, fruit juice, sugar solution, yogurt, sweetened or unsweetened condensed milk, juices, coffee or cereal beverage, chocolate beverage, nutritional oral supplement, gels or liquid supplements for sport etc. The powder according to the invention may be any of milk powder, dehydrated soup powder, shake powder or infant formulae, for example.

The hesperidin obtainable by the heat treatment processes of the invention has a smaller size distribution compared to commercially available hesperidin as illustrated in fig. 3. The raw data (not given) from which the PSD (particle size distribution) graphs were extracted show that in average 40% of the cumulative distribution of commercially available hesperidin particles have a size lower than 20 microns, while at least 80% of the cumulative distribution of hesperidin particles having undergone the processes of the invention show a size of less than 20 microns (Table 1 shows different % of cumulative distribution size obtained with different heat treatment conditions)

A process for improving the stability of flavanones in a liquid or semi-liquid food product comprising the steps of:
a. Heating said flavanone in water to a temperature of at least 138°C, preferably 140-160°C, for 1 to 60 seconds and
b. Incorporating said flavanone to the food product
or
heating said food product in the presence of said flavanone to a temperature of at least 138°C, preferably 138-160°C, most preferred 140-150°C for 1-60s is thus also part of the present invention. Preferably, the flavanone is a water insoluble flavanone. More preferably, the flavanone is hesperidin.

Food products obtainable by the processes of the invention also form part of the present invention.

The present invention relates, in a further aspect, to food products comprising flavanones, especially hesperidin. Commercially available hesperidin extract normally has a crystalline form wherein the crystals have a size distribution ranging from 0.1 to 160 microns and with an average of 40% of cumulative distribution of hesperidin particles below 20 microns.

The food products of the invention comprise flavanones in an amount of 0.05-5%, wherein the flavanone particles are obtained by heating said flavanone at a temperature of at least 138°C, preferably 138-160°C, most preferred 140-150°C for 1-60s Preferably, at least 80% of the cumulative distribution of flavanone particles has a size below 20 microns.

In an embodiment of the invention, the food product is in the form of a powder comprising flavanones, especially hesperidin, in an amount of 0.5-5%, preferably 0.5-3%, more preferably 0.5-1%. The powder can be obtained by a process of the invention followed by drying. Any method of drying known to a skilled person is suitable. The powder may be any of milk powder, dehydrated soup powder, shake powder or infant formulae, for example. In the most preferred embodiments of the invention, the food product is a milk powder or milk.

In another embodiment of the invention, the food product is in the form of a liquid or semi-liquid food product comprising flavanones, especially hesperidin, in an amount of 0.05-0.5%, preferably 0.05-0.3%, more preferably 0.05-0.1%. The food product is preferably selected from skimmed milk, partially skimmed milk, filled milk, fruit juice, sugar solution, yogurt, sweetened or unsweetened condensed milk, juices, coffee or cereal beverage, chocolate beverage, nutritional oral supplement, gels or liquid supplements for sport etc.

The present inventors have found that by incorporating hesperidin having at least 80% of the cumulative distribution of the particles having a size below 20 micron, wherein the flavanone particles are obtained by heating said flavanone at a temperature of at least 138°C, preferably 138-160°C, most preferred 140-150°C for 1-60s, a much more stable product can be obtained. Indeed, if the flavanone does not undergo the processes of the invention and thus the size and morphology changes, it is not stable in solutions and sedimentation occurs rapidly, forming brown sediments, thus altering the homogeneity and organoleptic properties of the product.

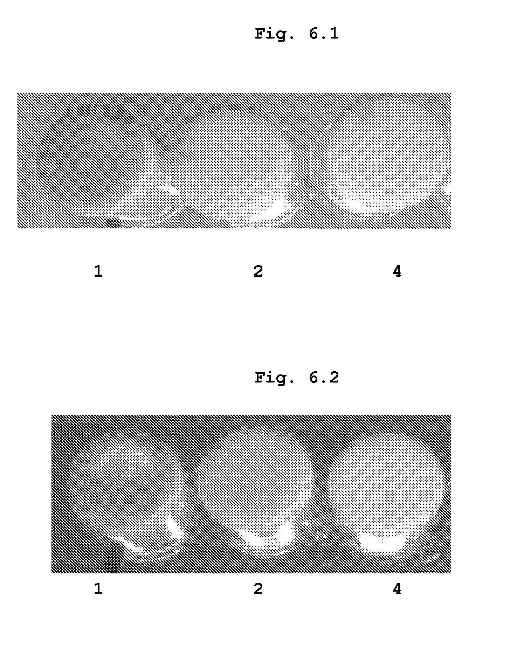

This is exemplified in figures 4 and 6, which show that in a reconstituted powder product or in a liquid milk product having commercially available hesperidin particles a dark brownish colour sediment forms already after 5 minutes of standing, whereas a reconstituted powder product or a liquid milk product containing hesperidin according to the invention maintains a "milk-like" colour and is indicative of good stability.

The organoleptic properties of the products of the invention are thereby improved. Thus, liquid products can be obtained which are stable over a period of time and do not alter due to hesperidin sedimentation.

The product according to the present invention may be for example a food product, a powder, a drink, a food supplement, a nutraceutical, a cosmetic product, a pet food product or a medicament. While the products are primarily intended to be used by humans, they may also be applied to animals, in particular companion animals, pets or livestock.

In an embodiment, the food products of the invention may be in the form of powders. The powders present the advantage that upon reconstitution in a liquid, the product remains stable for considerable amounts of time compared to products wherein the hesperidin has not undergone the processes of the invention. These powders may be easily reconstituted in a liquid such as water, juice, sugar solution, etc. Typically, 20-30 g of the powder may be mixed with 200-250mL of liquid to produce a drink according to the invention.

Thus, a process for preparing a liquid or semi-liquid food product, comprising the step of reconstituting the powders of the invention in a liquid such that the concentration of flavanone in the liquid or semi-liquid product is between 0.05-0.5%, preferably 0.05-0.3%, more preferably 0.05-0.1% also forms part of the invention.

The food products may be water and/or fat-based. They may comprise further ingredients such as fat, vitamins, minerals, probiotics, prebiotics, aromas, colorants, other functional ingredients etc. These may be incorporated at any stage during the processes of the invention.

According to the invention, the food products may be used for the manufacture of a composition for the improvement of bone and skin health.

The present invention thus presents the advantage that the processes for the manufacture of food products are simple and help control and reduce the hesperidin particle size. Stable and homogeneous liquid or semi-liquid products obtainable directly according to the invention or reconstituted from the powders of the invention ensue which provides a greater scope of application for hesperidin containing food products.

The present invention is illustrated below by means of non-limiting examples.

### Examples

### Example 1

In this example, the impact of heat treatment on hesperidin stability is described.

**Table 1.**

| **Variants** | **1** | **2** | **3** | **4** |
|---|---|---|---|---|
| **description** | water with 0.5% of commercially available hesperidin | water with 0.5% of commercially available hesperidin UHT treated at 140°C/10s | water with 0.5% of commercially available hesperidin UHT treated at 140°C/60s | water with 0.5% of commercially available hesperidin UHT treated at 160°C/10s |
| **preparation** | 0.5g of hesperidin in 99.5g water | 0.5g of hesperidin in 99.5g water UHT treated at 140°C/10s | 0.5g of hesperidin in 99.5g water UHT treated at 140°C/60s | 0.5g of hesperidin in 99.5g water UHT treated at 160°C/10s |
| **visual results** | brown sediment appears at the bottom of glass after few minutes (2-3 minutes) | opaque suspension | opaque suspension | opaque suspension |
| **% of cumulative distribution of hesperidin particle below 20 microns** | Average 40% | 86% | 99% | 99% |

Fig. 2.1, 2.2, 2.3 and 2.4 show the microscopic images of hesperidin according to preparation variants 1, 2, 3 and 4 respectively.

Fig. 3.1, 3.2, 3.3 and 3.4 shows the particle size distribution of hesperidin prepared according to preparation variants 1, 2, 3, and 4 respectively.

The raw data from which the particle size distribution (PSD) graph in fig.3.1 to 3.4 have been extracted show that for variant 1 (raw hesperidin) the cumulative distribution of particle size below 20 microns is 40% (average) whereas for heat treated hesperidin according to the invention (140°C, 10s) the cumulative distribution of particle size below 20 microns is 86%. For variant 3 (140°C, 60s) and variant 4 (160°C, 10s), the cumulative distribution of particle size below 20 microns increases to 99%. Thus, it can be seen that the heat treatment has an influence on the particle size of hesperidin and on the conformation of hesperidin particles. Therefore, sedimentation is avoided.

### Example 2:

A formulation of a liquid UHT skimmed milk containing 0.1% hesperidin is presented below:

**Table 2.**

| | **Example of a formulation to produce a UHT skimmed milk containing 0.1% hesperidin** |
|---|---|
| Ingredients | Batch load |
| MSK | 9.3 |
| Water | 77.4 |
| Total MSK solution | 86.70 |
| | |
| CMC/MCC* | 0.2 |
| Hesperidin | 0.1 |
| Water | 13.2 |
| Total hesperidin mix | 13.3 |
| **Total** | 100.00 |

| | |
|---|---|
| MSK: skimmed milk solids * Carboxy Methyl Cellulose/ Microcrystalline Cellulose | |

A process according to the invention to produce a liquid UHT skimmed milk containing 0.1 % hesperidin is described below:
The skimmed milk is dissolved in 50°C water for 20 min in a tank using a high shear mixer. In parallel, the hesperidin solution is prepared by adding commercially available hesperidin powder and the stabilising agent (CMC/MCC) into water (20°C) to reach a dry matter composition of 1 to 3%. The mix is stirred using a high shear mixer for 15min. The hesperidin mix is then incorporated into the milk and further mixed for another 5 min. The milk is then standardised to pH between 6.4 to 6.9 and to dry matter between 9% DM to 10%DM. The mix is preheated to 70°C using plate heat exchanger, and further UHT treated at temperature of 140°C for 5s. The mixed is then passed through an aseptic homogeniser with a pressure of 180bar 1st stage and 40 bars 2nd stage. After homogenisation, the mix is cooled to 20°C prior to being filled in tetra packs.

The stability of the product is measured every month until 12 months by opening the packaging from the top and pouring the liquid. The bottom of the pack in then observed and compared to an existing scale (from 0: no sediment to 5: heavy sediment).

Another method used to control the stability of hesperidin in the product is to pour the liquid part into a container and measure the amount of hesperidin into the liquid knowing the quantity of liquid. The amount of hesperidin which sedimented on the bottom of the pack can then be calculated. According to our measurements, after 12 months, no sedimentation of hesperidin was observed in the milk product according to the invention.

### Example 3

This example compares the stability of hesperidin in liquid milk containing stabilising agents (Carboxy Methyl Cellulose/ Microcrystalline Cellulose) with and without heat treatment according to the invention. The mode of preparation of samples 1 to 4 is described in the table below.

**Table 3**

| **Variants** | **1** | **2** | **3** | **4** |
|---|---|---|---|---|
| description | liquid skimmed milk with stabilising agent (CMC/MCC) 0.2% UHT treated + 0.1% hesperidin | liquid skimmed milk with stabilising agent (CMC/MCC) 0.2% + hesperidin (0.1%) heat treated | liquid skimmed stabilising agent (CMC/MCC) 0.2% UHT treated + 0.5% hesperidin | liquid skimmed milk with stabilising agent (CMC/MCC) 0.2% + hesperidin (0.5%) heat treated |
| preparation add | pour 200ml pack of the liquid skimmed milk UHT treated and 0.1% of hespendin. Mix and follow stability | pour 200ml pack of liquid skimmed milk UHT treated already containing 0.1%hesperidin and follow stability | pour 200ml pack of the liquid skimmed milk UHT treated and add 0.5% of hespendin. Mix and follow stability | pour 200ml pack of liquid skimmed milk UHT treated already containing 0.5% hesperidin and follow stability |
| Results | Brown sediment appears at the bottom of glass after few minutes and is clearly visible after 3-5 min. | No sediment is visible even after 12h | Brown sediment appears at the bottom of glass after few minutes and is clearly visible after 3-5 min. | No sediment is visible even after 12h |

Fig. 4.1 and 4.2 show the visual results of variants 1, 2, 3, 4 after 5 minutes and after 12 hours respectively.

### Example 4

An example of a formulation of a skimmed milk powder containing 1% hesperidin according to the invention is presented below:

**Table 4**

| **Ingredients** | **Batch load (Kg)** |
|---|---|
| Hesperidin powder | 1 |
| MSK | 96 |
| water | 862 |
| *Total solution before UHT treatment* | *960* |
| | |
| *Total after spray drying* | 100 |

After the heat treatment (e.g. 140°C for 10s) and the homogenisation step, the mix is evaporated to 40%TS using an evaporator and further spray dried to obtain a powder of about 96% TS.

Upon reconstitution in water, the solution showed improved stability compared to a reconstituted milk powder comprising hesperidin which has not been heat-treated.

### Example 5

This example compares the stability of hesperidin in reconstituted milk (variant 1) compared to the stability of dry milled hesperidin in reconstituted milk (variant 2) and compared to the stability of heat treated hesperidin in reconstituted milk (variant 4).

**Table 5**

| **Variants** | **1** | **2** | **4** |
|---|---|---|---|
| description | MSK powder + 1% standard hesperidin reconstituted in water | MSK powder + 1% hesperidin dry milled reconstituted in water | heat treated hesperidin and water solution at 0.1 % hesperidin used to reconstitute MSK |
| % of cumulative distribution < 20 microns of hesperidin in water | Standard hesperidin average 40% | Dry-milled hesperidin 99% | Heat treated hesperidin 86% |
| preparation | Mix 20g MSK with 0.2g hesperidin standard and add 200ml of 40 deg C water. Mix and follow stability | Mix 20g MSK with 0.2g hesperidin dried mill and add 200ml of 40 deg C water. Mix and follow stability | 200ml water containing 0.1% heat treated hesperidin heated to 40deg C and mix in 20g MSK. Mix and follow stability |
| Results | Brown sediment appears at the bottom appears at the bottom of glass after few minutes and is clearly visible after 5 min. | light brown sediment is visible after 5 minutes visible | No sediment is even after 12h |

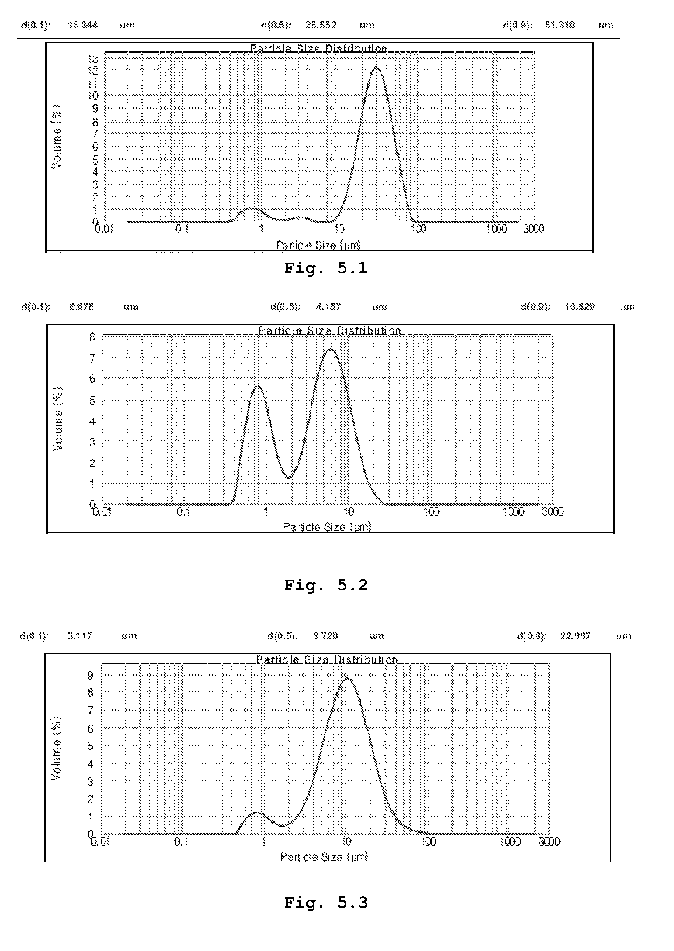

Figures 5.1, 5.2 and 5.3 show the particle size distribution of variants 1, 2 and 4 respectively. A comparable reduction of particle size can be observed in the dry milled hesperidin and the heat treated hesperidin according to the invention.

Figure 6.1 and 6.2 show the impact of the different hesperidin forms on the stability of the reconstituted milk after 5 minutes and 12 hours respectively.

The dry-milling of hesperidin (with 99% of cumulative distribution below 20 microns) leads to a reduction of the sedimentation and gives a lighter sediment colour but does not prevent sedimentation as for heat treated hesperidin at 0.1% hesperidin concentration in final product. This shows that despite the smaller particle size distribution results of the dry milled hesperidin vs. the heat treated hesperidin, the stability of hesperidin in liquid milk or reconstituted powder milk is still better with heat treated hesperidin than dry milled hesperidin (with 99% of cumulative distribution below 20 microns).

## Claims

1. Process for the manufacture of a powder, liquid or semi-liquid food product comprising flavanones, comprising the steps of:
a. Heating said flavanone in water to a temperature of at least 138°C, preferably 140-160°C, for 1 to 60 seconds and
b. Incorporating said flavanone to the food product.

2. Process for the manufacture of a powder, liquid or semi-liquid food product comprising flavanones, comprising the step of heating a liquid or semi-liquid food product comprising said flavanone in an amount of 0.05-5% to a temperature of at least 138°C, preferably 138-160°C, most preferred 140-150°C for 1-60s.

3. Process according to any of the preceding claims, comprising further processing steps selected from any of pH adjustment, homogenisation, pasteurisation, preheating, cooling, filling, drying, spray-drying.

4. Process according to any of the preceding claims, wherein the food product is any of a powder such as milk powder, dehydrated soup powder, shake powder, infant formulae or a liquid or semi-liquid food product such as skimmed milk, partially skimmed milk, full milk, filled milk, fruit juice, sugar solution, yogurt, sweetened or unsweetened condensed milk, juices, coffee or cereal beverage, chocolate beverage, nutritional oral supplement, soup, shake, gels or liquid supplements for sport.

5. Food product obtainable by a process according to any of the preceding claims.

6. Process for improving the stability of flavanones in a liquid or semi-liquid food product comprising the steps of:
a. Heating said flavanone in water to a temperature of at least 138°C, preferably 140-160°C, for 1 to 60 seconds and
b. Incorporating said flavanone to the food product
or
heating said food product in the presence of said flavanone to a temperature of at least 138°C, preferably 138-160°C, most preferred 140-150°C for 1-60s.

7. Process according to any of the preceding claims, wherein the flavanone is hesperidin.

8. Food product comprising flavanones in an amount of 0.05-5%, wherein the flavanone particles are obtained by heating said flavanone at a temperature of at least 138°C, preferably 138-160°C, most preferred 140-150°C for 1-60s

9. Food product according to claim 8, which is in the powder form, preferably selected from milk powder, dehydrated soup powder, shake powder, infant formulae, and wherein the amount of flavanones is 0.5-5%, preferably 0.5-3%, more preferably 0.5-1%.

10. Food product according to claim 8, which is in the liquid or semi-liquid form, preferably selected from skimmed milk, partially skimmed milk, full milk, filled milk, fruit juice, sugar solution, yogurt, sweetened or unsweetened condensed milk, juices, coffee or cereal beverage, chocolate beverage, nutritional oral supplement, soup, shake, gels or liquid supplements for sport, and wherein the amount of flavanones is 0.05-0.5%, preferably 0.05-0.3%, more preferably 0.05-0.1 %.

11. Food product according to any of claims 8 to 10, wherein the food product is a food supplement, a nutraceutical, a cosmetic product, a pet food product or a medicament.

12. Use of a food product according to any of claims 8 to 11, for the manufacture of a composition for the improvement of bone and skin health.

13. Process for preparing a liquid or semi-liquid food product, comprising the step of reconstituting the food product of claim 9 in a liquid such that the concentration of flavanone in the liquid or semi-liquid product is between 0.05-0.5%, preferably 0.05-0.3%, more preferably 0.05-0.1%.

14. Process for producing stabilised flavanones comprising the step of heating said flavanone in water to a temperature of at least 138°C, preferably 140-160°C, for 1 to 60 seconds.

15. Flavanone obtainable by the process of claim 14.
